# EUROPEAN PATENT APPLICATION

(11) **EP 4 316 257 A1**
(43) Date of publication of application: **07.02.2024**
(21) Application number: 22780445.7
(22) Date of filing: 24.03.2022
(51) Int. Cl.: A23J 3/08, A23C 21/00, A23L 33/19

(54) **PRODUCTION METHOD FOR FOOD COMPOSITION**

(30) Priority: 30.03.2021 JP 2021056998
(71) Applicant: Megmilk Snow Brand Co. Ltd., Sapporo-shi, Hokkaido 065-0043 (JP)
(72) Inventor: FUKUDOME Hirofumi, Sapporo-shi, Hokkaido 065-0043 (JP); SHIBA Mayumi, Sapporo-shi, Hokkaido 065-0043 (JP); TAKEMOTO Atsuhiro, Sapporo-shi, Hokkaido 065-0043 (JP); SAKAI Fumihiko, Sapporo-shi, Hokkaido 065-0043 (JP)
(74) Representative: Forstmeyer, Dietmar
(86) International application number: PCT/JP2022/013811
(87) International publication number: WO 2022/210231

(57) **Abstract**

A production method for an MP whey is provided, that enables preparation of both of a high GMP-content composition and a usable MP whey from an ordinary MP whey. The problems can be solved by a production method for a glycomacropeptide-containing composition, that includes a step of acquiring a solution containing glycomacropeptide, that includes a glycomacropeptide content of 10% by weight or higher in the protein total amount by condensing and/or applying diafiltration to a microparticulated whey solution.

## Description

### TECHNICAL FIELD

The present invention relates to a production method for a glycomacropeptide-containing composition. The present invention also relates to a production method for a glycomacropeptide-containing food composition. The present invention further relates to a microparticulated whey composition for producing the glycomacropeptide-containing composition.

### BACKGROUND ART

κ-casein glycomacropeptide included in cheese whey and rennet whey is also referred to as "glycomacropeptide (hereinafter, may be referred to as "GMP")". GMP is a sialic acid-binding peptide produced when rennet or pepsine is caused to act on κ-casein of bovine milk.

Human milk includes sialic acid in a three-fold to a five-fold amount compared to that of bovine milk, and sialic acid assumes protection against infection, promotion of cerebral development, the promoting activity on proliferation of bifidobacteria, and the like for babies. It is known that GMP including sialic acid also has these functionalities. Industrial-scale production of GMP is therefore strongly desired to use GMP in replacements for human milk, functional foods, and the like. Methods each using ultrafiltration, a reverse osmosis membrane, or an ion-exchanger resin have been disclosed so far as a technique to separate GMP from milk.

Patent Document 1 discloses a production method for GMP, that is characterized in that (1)pH of milk-quality whey including GMP is adjusted to be lower than 4 and a filtrate is thereafter acquired from the whey by an ultrafiltration membrane process with a molecular weight cut off of 10,000 Da to 50,000 Da to condense the filtrate using a membrane whose molecular weight cut off is 50,000 Da or lower. Patent Document 1 also discloses a method for acquiring a composition having a high GMP content by, after readjusting pH of the filtrate acquired in (1) to be pH4 or higher, condensing the filtrate using an ultrafiltration membrane whose molecular weigh cut off is 50,000 Da or lower, and by condensing the filtrate acquired in (1) using an ultrafiltration membrane whose molecular weight cut off is 10,000 Da or lower.

Patent Document 2 discloses a production method for a composition having a high GMP content, that is characterized in that pH of milk-quality whey is adjusted to have pH of 5.0 or higher and heat treatment is applied to the milk-quality whey at a temperature of 80°C or higher to cause a milk serum protein association body to be generated, the generated associated body is separated using a microfiltration membrane whose pore size is 0.5 µm or smaller, or an ultrafiltration membrane whose molecular weight cut off is 50,000 Da or higher, and GMP is collected in the permeate.

Patent Document 3 discloses a method for producing a GMP-containing composition, that includes a step of providing a whey-origin supply that includes GMP and at least one type of additional protein and whose pH is 4 at the highest, a step of applying ultrafiltration to the whey-origin supply using an ultrafiltration membrane filter that causes monomer GMP to permeate therethrough and thereby providing a UF permeate whose GMP is enriched and the UF residue part, a step of bringing a first composition originated from the UF permeate into contact with a cation exchange material, and a step of collecting a fraction of the first composition, that does not bond with the cation exchange material and thereby acquiring the GMP-containing composition.

Patent Document 4 discloses a method according to which an aqueous solution of a milk raw material that includes GMP and whose pH is 4.0 to 9.0 is prepared, hydrogen carbonate ions and/or carbonate ions are generated in the aqueous solution, and a composition including GMP is thereby acquired from a permeate acquired by processing the aqueous solution with a membrane whose molecular weight cut off is 9,000 Da to 50,000 Da even though pH is 4.0 or higher.

### CITATION LIST

### PATENT LITERATURE

Patent Document 1: JP H02-276542A
Patent Document 2: JP H05-271295A
Patent Document 3: JP 2015534832 A
Patent Document 4: WO 2019/189350 A

### SUMMARY OF INVENTION

### TECHNICAL PROBLEM

With the techniques disclosed by Patent Documents 1, 3, and 4, however, problems arise that the number of necessary steps is great, that the operation is complicated, and that a long time and a lot of labor are necessary to acquire the aimed substance. With the technique disclosed by Patent Document 2, only the heating treatment is applied to the whey at 80°C or higher and proteins other than GMP are therefore denatured to have aggregation and gelation occurring therein. The aggregated or gelated whey protein is difficult to handle in the processing treatment executed thereafter. This therefore not only is an industrial demerit but also significantly influences the taste and the texture of foods. Even through the whey protein and GMP are separated from each other using this method, the remaining whey protein therefore has a low utility value and is often discarded. As a result, problems arise that a large food loss is created and that the production cost of GMP is high.

On the other hand, a technique is recently prevalent as a method for improving the thermal stability of whey protein, according to which a heating shear process is applied to whey and the whey is thereby processed into the state of fine particles that are excellent in the dispersibility and that have a weak reaggregation property. The whey undergoing the above processing treatment is referred to as "microparticulated whey (hereinafter, may be referred to as "MP whey")". The MP whey is expected to be applied to various milk products because of its high thermal stability and its high dispersibility. On the other hand, any technique of separating GMP from the MP whey has not been reported so far.

### SOLUTION TO PROBLEM

The present invention provides a production method for a GMP-containing composition, according to which a composition including GMP is acquired by condensing and/or applying diafiltration to the MP whey that is prepared by heating and applying a shear process to whey.

The present invention employs the following configuration.

<1> A production method for a glycomacropeptide-containing composition, comprising
   condensing and/or applying diafiltration to a microparticulated whey solution, and
   acquiring a solution containing glycomacropeptide,
   wherein a glycomacropeptide content in the protein total amount is 10% by weight or higher.
<2> The production method for a glycomacropeptide-containing composition described in <1>, wherein
   the condensation and/or the diafiltration is/are each a microfiltration membrane process, an ultrafiltration membrane process, an ion exchanging membrane process, or centrifugal separation.
<3> The production method for a glycomacropeptide-containing composition described in <2>, wherein
   the condensation and/or the diafiltration is/are each a microfiltration membrane process or an ultrafiltration membrane process,
   the membrane is a microfiltration membrane whose pore size is 10 µm or smaller, or an ultrafiltration membrane whose molecular weight cut off is 50,000 Da or higher, and
   a glycomacropeptide-containing composition is acquired as a permeate.
<4> The production method for a glycomacropeptide-containing composition described in any one of <1> to <3>, further comprising
   acquiring a microparticulated whey solution by heating and applying a shear process to a whey protein solution.
<5> The production method described in any one of <1> to <4>,wherein
   the volumetric median diameter (d50) of the microparticulated whey is 0.1 µm to 100 µm.
<6> The production method for a glycomacropeptide-containing composition described in any one of <1> to <5>, further comprising
   removing lactose and/or minerals by recondensing, applying rediafiltration to, and/or demineralizing (desalting) the solution containing glycomacropeptide.
<7> The production method for a glycomacropeptide-containing composition described in <6>, wherein
   the removal of the lactose and/or the minerals is executed using an ultrafiltration membrane whose molecular weight cut off is 10,000 Da or lower, or vacuum concentration.
<8> A production method for a glycomacropeptide-containing composition, comprising
   condensing, applying diafiltration to, and/or demineralizing the composition acquired by the production method for a glycomacropeptide-containing composition described in any one of <1> to <7>, and
   removing lactose and minerals from the composition.
<9> A microparticulated whey composition for producing a glycomacropeptide-containing composition.
<10> A production method for a glycomacropeptide-containing food composition, comprising:
   producing a glycomacropeptide-containing composition by the production method for a glycomacropeptide-containing composition described in any one of <1> to <8>; and
   adding the glycomacropeptide-containing composition to a food.
<11> The production method described in any one of <1> to <8>, wherein
   the volumetric median diameter (d50) of the microparticulated whey is 0.1 µm to 5 µm,
<12> The production method described in any one of <4> to <8>, wherein
   the heating and the shearing are executed until the volumetric median diameter (d50) of the microparticulated whey becomes 0.1 µm to 100 µm,
<13> The production method described in <12>, wherein
   the time period of the heating is 4 minutes or longer at 70°C to 150°C.
<14> The production method described in any one of <4> to <13>, wherein
   pH of a whey protein solution to undergo the heating and the shear process is 4.0 to 6.5.
<15> The production method described in <14>, wherein
   pH of the whey protein solution to undergo the heating and the shear process is 5.0 to 6.2.
<16> The production method described in any one of <3> to <15>, wherein
   the membrane is a microfiltration membrane whose pore size is 0.1 µm or smaller, or an ultrafiltration membrane whose molecular weight cut off is 50,000 Da or higher,.
<17> The production method described in any one of <1> to <16>, wherein
   a glycomacropeptide content of the solution containing glycomacropeptide is 40% by weight or higher.
<18> A microparticulated whey composition for producing a glycomacropeptide-containing composition.
<19> The microparticulated whey composition described in <18>, wherein
   the content of microparticulated whey is 10% by weight or higher based on the total amount of the composition.
<20> The microparticulated whey composition described in <18> or <19>, wherein
   the content of the microparticulated whey is 10% by weight or higher in the protein total amount.
<21> A separation method for microparticulated whey and a glycomacropeptide-containing composition from each other, comprising
   acquiring a solution that contains glycomacropeptide by condensing and/or applying diafiltration to a microparticulated whey solution.

### ADVANTAGEOUS EFFECTS OF INVENTION

According to the present invention, the production method for the novel high-GMP content composition is provided. The material including a large amount of GMP, that is acquired by the production method has an infection protection action against viruses and bacteria, has a bifidobacteria proliferative activity, and/or achieves a brain function improving effect. This material is therefore useful as a means of maintaining and improving the health functions, and is advantageously usable in medical drugs, foods, and beverages. The MP whey-containing solution that is collected in the condensed portion by the membrane process is not different compared to an ordinary MP whey-containing solution to which no membrane process is applied except the fact that the collected solution has an increased protein content. The MP whey-containing solution collected in the condensed portion is therefore usable in various foods as an ordinary MP whey.

In the case where water is added to the MP whey-containing solution in an amount equal to the amount thereof and diafiltration is applied to the resultant solution using, for example, an ultrafiltration membrane, the liquid amounts of the retentate and the permeate of the diafiltration are equal to each other. The retentate of the diafiltration includes materials such as carbonhydrates and minerals each in a half amount compared to that of the MP whey-containing solution while the retentate is usable in various foods as an MP whey. The permeate includes GMP.

As above, both of the high-GMP content composition and the usable MP whey can be prepared from an ordinary MP whey-containing solution. As a result, the production cost of the prepared high-GMP content composition can be reduced.

In the case where GMP is separated from the whey protein, the used whey protein has a degraded quality due to the denaturing by the heating and may be discarded. Otherwise, even when the used whey protein is usable, the uses thereof have been strictly limited. GMP could also have been separated without heating the whey protein while, in this case, the steps are complicated and a large amount of labor was necessary. The production yield was highly likely to be reduced due to the great number of the steps. The present invention solves these problems.

### DESCRIPTION OF EMBODIMENTS

The present invention will be described below in detail. The description will be made herein for each of the aspects of the invention while the items, the definition of a word, and an embodiment that are described in each of the aspects are applicable to other aspects.

### (Glycomacropeptide )

Glycomacropeptide (GMP) is a C-terminal peptide of κ-casein that includes a sugar chain and, in the case of bovine milk, corresponds to the 106 to 169 residues of x-casein. The molecular weight of GMP is 7,000 Da to 9,000 Da. GMP is generated when rennet or pepsine is caused to act on κ-casein of bovine milk.

### (Microparticulated whey)

"Microparticulated whey (MP whey)" as used herein means whey protein that is pulverized into microparticles by heating and applying a shear process to a whey protein solution. The "MP whey" as used herein is an aggregate of the whey protein whose volumetric median diameter (d50) is 0.1 µm to 100 µm. The MP whey is pulverized into microparticles and the particle diameter thereof is larger than that of an ordinary whey protein solution. The volumetric median diameter (d50) of the MP whey used in the present invention is preferably 0.1 µm to 50 µm, more preferably 0.1 µm to 10 µm, further preferably 0.1 µm to 5 µm, and most preferably 0.1 µm to 3 µm when the median diameter is measured by a laser diffraction-type particle size distribution measuring apparatus.

The "whey protein" as used herein means the protein included in whey. Typical whey protein components include β-lactoglobulin, α-lactalbumin, and immune globulin.

Whey protein included in cheese whey or rennet casein whey that is acquired in the course of producing a milk product such as cheese may be used, or whey protein included in a whey protein raw material such as a whey protein concentrate (WPC) or a whey protein isolate (WPI) may also be used, as the whey protein to prepare the MP whey. It is preferred that cheese whey or rennet casein whey acquired in the course of producing a milk product such as cheese be used.

The WPC is a substance produced by separating lactose, minerals, and vitamins from whey, and collecting and powdering the whey protein. The WPC is produced by processing a milk-origin solution that includes the whey protein such as cheese whey and acid whey using a filtration membrane such as an ultrafiltration membrane or a diafiltration membrane and thereby condensing and drying the whey protein. The WPI is produced at the same steps as those for the WPC while the WPI is a substance acquired by condensing and separating only the protein in the whey by an ion exchange chromatography or the like, and generally has a higher protein content than that of the WPC. A WPC and a WPI that are prepared by the user may be used or those commercially available can also be used.

The MP whey is excellent in dispersibility and has weak reaggregation properties, and is therefore expected to be applied to various processed foods. Any technique of separating GMP from the MP whey has, however, not yet been reported.

The MP whey may be prepared by the user or a commercially available MP whey may be purchased to be used. For example, Y0-8075 (Aria Foods amba) or WPC550 (Fonterra Co-operative Group Limited) can be used as the commercially available MP whey. In the case where the commercially available MP whey is used, it is preferred that homogenization be executed therefor to further reduce the volumetric median diameter (d50) thereof. Mineral whey may be added to the commercially available MP whey to supply minerals and the like. The volumetric median diameter (d50) of the MP whey solution after the homogenization can be 0.1 µm to 100 µm, 0.1 µm to 50 µm, 0.1 µm to 10 µm, 0.1 µm to 5 µm, or 0.1 µm to 3 µm. A commercially available homogenizer by SPX Corporation or the like can be used for the homogenization.

It is preferred that an MP whey prepared by the user be used as the MP whey. The case where the MP whey prepared by the user is used will be described below.

An MP whey solution can be acquired by heating and applying a shear process to a whey protein solution. The temperature and the time period of the heating are not limited as long as the whey protein can be pulverized into microparticles while the temperature and the time period are, for example, 70°C or higher and 4 minutes or longer, preferably 70°C or higher and 5 minutes or longer, more preferably 75°C or higher and 4 minutes or longer, further preferably 75°C or higher and 5 minutes or longer, further preferably 80°C or higher and 4 minutes or longer, further preferably 80°C or higher and 5 minutes or longer, further preferably 85°C or higher and 4 minutes or longer, further preferably 85°C or higher and 5 minutes or longer, further preferably 90°C or higher and 4 minutes or longer, or most preferably 90°C or higher and 5 minutes or longer. The upper limit of the heating temperature can be 150°C. The upper limit of the heating time period can be 30 minutes.

A known heating means such as, for example, a plate-type heat exchanger or a tube-type heat exchanger is usable as the heating means.

The whey protein solution can be heated and undergo the shear process such that the volumetric median diameter (d50) of the MP whey solution is 0.1 to 100 µm, preferably 0.1 µm to 50 µm, more preferably 0.1 µm to 10 µm, further preferably 0.1 µm to 5 µm, and most preferably 0.1 µm to 3 µm.

pH of the whey protein solution to undergo the heating and the shear process is, for example, 4.0 to 6.5, 4.5 to 6.2, 5.0 to 6.2, or 5.5 to 6.2. The method for adjusting pH of the whey protein solution is not limited as long as the whey protein can be pulverized into microparticles, while examples thereof include a method normally employed in the art such as, for example, adjustment by adding a lactic acid aqueous solution to the whey protein solution.

The protein content of the whey protein solution to undergo the heating and the shear process is, for example, 5% by weight or higher, or 10% by weight to 20% by weight based on the whey protein solution.

The whey protein solution to undergo the heating and the shear process includes lactose at, for example, 1% by weight or higher, or 3% by weight or higher based on the whey protein solution.

The whey protein solution to undergo the heating and the shear process includes lipid at 1% by weight or lower, or 0.5% by weight or lower based on the whey protein solution.

The shear process can be executed at the above heating temperature. The means of executing the shear process is not limited as long as the whey protein can be pulverized into microparticles. For example, a mixer or a homogenizer is usable.

A production method for a glycomacropeptide-containing composition of the present invention can include a step of solving crystals of lactose in the whey protein solution, before executing the heating and the shear process. Any damage caused by the crystals of lactose to the apparatus or the membrane used for the condensation can be prevented by including the above step. The step of solving the crystals of lactose is a step at which the whey protein solution is maintained at preferably 40°C for 15 minutes or longer, preferably 50°C for 15 minutes or longer, and further preferably 50°C for 20 minutes or longer.

The production method for a glycomacropeptide-containing composition of the present invention can include a step of removing casein and lipid in the whey protein solution before executing the heating and the shear process. The step of removing casein and lipid is preferably a step at which the whey protein solution is processed by a clarifier or a separator.

The production method for a glycomacropeptide-containing composition of the present invention can include a step of removing lactose, amino acids, minerals, and the like in the whey protein solution before executing the heating and the shear process. The concentration of the whey protein in the whey protein solution can be increased and the production efficiency of the MP whey can be increased by including the above step. The step of removing lactose, amino acids, minerals, and the like is preferably a step at which the whey protein solution is filtered using an ultrafiltration membrane whose molecular weight cut off is 10,000 Da or lower.

### (Concentration of Microparticulated whey Solution)

The protein concentration of the MP whey solution set when the solution undergoes condensation and/or diafiltration is, for example, 5% by weight or higher, or 10% by weight to 20% by weight.

### (Condensation and/or Diafiltration)

In the production method for a glycomacropeptide-containing composition of the present invention, the MP whey solution undergoes concentration and/or diafiltration to acquire a solution that includes glycomacropeptide. The "condensation" of the MP whey solution as used herein means an operation of removing water and other components from the MP whey solution to acquire the MP whey solution and the glycomacropeptide-containing composition. The "diafiltration" of the MP whey solution as used herein means an operation of adding a liquid including no MP whey (such as water, the permeate by a membrane, or the like) to the MP whey solution and removing, thereafter or during the addition, water and the other components from the microparticulated whey solution to acquire the MP whey solution and the glycomacropeptide-containing composition.

Glycomacropeptide may be included in the MP whey solution or may be included in the removed water and the removed other components. The condensation and/or the diafiltration are not especially limited as long as the glycomacropeptide-containing composition can be acquired while the condensation and the diafiltration can be executed by a microfiltration membrane process, an ultrafiltration membrane process, an ion exchanging membrane process, or centrifugal separation but it is preferred that the condensation and the diafiltration be executed by a microfiltration membrane process or an ultrafiltration membrane process. In the case where the condensation and the diafiltration are executed by a microfiltration membrane process or an ultrafiltration membrane process, a solution containing glycomacropeptide can be acquired as the permeate. A condensed liquid of the MP whey or a retentate of the diafiltration can be acquired as the residual liquid (a condensed liquid, a retentate of the diafiltration). The condensed liquid of the MP whey or the retentate of the diafiltration is not different from the ordinary MP whey except its smaller content of glycomacropeptide, and is usable for any use.

The membrane of the membrane process is not limited as long as a solution containing glycomacropeptide can be acquired using the membrane while it is preferred that the membrane be a microfiltration membrane whose pore size is 10 µm or smaller, or an ultrafiltration membrane whose molecular weight cut off is 50,000 Da or higher. The "molecular weight cut off" as used herein means an approximate molecular weight of the globular molecules at a low concentration whose 90% are retainable.

It can be considered that the particle diameter varies in response to the heating and the shear process executed for the whey protein solution. The membrane having the pore size or the molecular weight cut off corresponding to the particle diameter of the MP whey can therefore be used, and a microfiltration membrane whose pore size is, for example, 5 µm or smaller, 1 µm or smaller, or 0.1 µm or smaller can be used.

The solution that is acquired after the condensation and/or the diafiltration, and that includes glycomacropeptide has a glycomacropeptide content of 10% by weight or higher, more preferably 20% by weight or higher, further preferably 30% by weight or higher, further preferably 40% by weight or higher, further preferably 50% by weight or higher, further preferably 60% by weight or higher, further preferably 70% by weight or higher, and most preferably 80% by weight or higher. The MP whey solution may undergo further condensation and/or a further diafiltration process after undergoing the condensation and/or the diafiltration, and the glycomacropeptide content in the solution containing glycomacropeptide only has to finally become 10% by weight or higher.

In the case where the MP whey solution is condensed by centrifugal separation, the centrifugal separation can be executed at the revolution number that is not limited to the followings while the centrifugal separation can be executed at, for example, 6,000 rpm or higher. A centrifugal separator that is commercially available such as a milk separator manufactured by Saito Separator Limited is usable as the centrifugal separator. The MP whey solution is separated into a heavy liquid and a light liquid. The glycomacropeptide-containing composition can be acquired by recondensing or drying the light liquid as necessary. The heavy liquid is not different from the ordinary MP whey except its smaller content of glycomacropeptide, and is usable for any use.

### (Recondensation, Rediafiltration, and/or Demineralization)

It is preferred that the production method for a glycomacropeptide-containing composition of the present invention include a step of removing lactose and/or minerals by executing the recondensation (i.e., condensing again), the rediafiltration (i.e., diafiltrating again), and/or demineralization for the glycomacropeptide-containing composition. The glycomacropeptide content in the composition can be increased by including this step, and distribution thereof and addition thereof to foods can be facilitated. The recondensation, the rediafiltration, and/or the demineralization can be executed by a microfiltration membrane process, an ultrafiltration membrane process, an ion exchanging membrane process, vacuum concentration, centrifugal separation, or the like, and are executed by preferably an ultrafiltration membrane whose molecular weight cut off is 10,000 or lower, or vacuum concentration.

### (Glycomacropeptide-Containing Composition)

The glycomacropeptide-containing composition produced by the production method for a glycomacropeptide-containing composition of the present invention has a glycomacropeptide content of 10% by weight or higher, more preferably 20% by weight or higher, further preferably 30% by weight or higher, further preferably 40% by weight or higher, further preferably 50% by weight or higher, further preferably 60% by weight or higher, further preferably 70% by weight or higher, and most preferably 80% by weight or higher, in the total amount of protein. "In the total amount of protein" means that the full amount of the included protein is used as the criterion.

The content of the protein in the glycomacropeptide-containing composition can be checked using an improved Duma's method (combustion method). One example of the procedure therefor will be described below.

The nitrogen content is determined using the improved Duma's method (combustion method) using TruSpecN (LECO Corporation). The operation method for the apparatus follows the instruction manual produced by LECO Corporation. The amount of the protein is acquired by multiplying the measured nitrogen content by a protein conversion factor. It is assumed that the protein conversion factor is 6.38.

The content of the glycomacropeptide in the protein can be checked by high performance liquid chromatography (HPLC). One example of the procedure therefor will be described below.

Freeze-dried samples are adjusted to be 10.5 and 2.5 mg/mL using a 40%-acetonitrile solution containing 0.1%-trifluoroacetic acid that is the mobile phase, to acquire sample solutions. The insoluble matters in the sample solutions are removed using a filter (Kurabo Industries Ltd., 0.45 µm, 13P, for both of an aqueous system and a non-aqueous system). An L-2000 (HITACHI) system having 2×TSKgel G3000PW (Tosoh Corporation) attached thereto is used for the HPLC analysis, and measurement is executed using a UV detector (210 nm). Elution is executed at a flow rate of 0.3 mL/min at 28°C for 120 minutes using a 40%-acetonitrile solution containing 0.1%-trifluoroacetic acid as the mobile phase. To quantify β-lactoglobulin (β-Lg) and α-lactalbumin (α-La), standard preparations (both from SIGMA Corporation) are used that are commercially available as reagents. To quantify GMP, a commercially available high GMP-content material (Aria Foods amba, CGMP-10) is used. CGMP-10 has a GMP content at about 60% in the solid and using the value as the criterion for the GMP content the quantification of GMP in each of the samples is therefore executed. A calibration curve is produced to quantify the GMP amount in each of the specimens.

The production method for a glycomacropeptide-containing composition of the present invention can include a step of spray-drying the solution that includes glycomacropeptide acquired by the condensation and/or the diafiltration.

It is preferred that a method of drying the solution containing glycomacropeptide using a spray-drying method be used as the method of powdering the raw material liquid. In the spray-drying method, for example, the raw material liquid is sprayed by a spray dryer to be hot-air-dried. The raw material liquid may be dried using a method other than the spray-drying method and the raw material liquid may be powdered by pulverizing, or the like, the acquired solid material.

The glycomacropeptide-containing composition produced using the production method for a glycomacropeptide-containing composition of the present invention can take any optional form and can take, for example, a liquid form, a solid form, a powder form, a granular form, or a gel form.

### (Production Method for Glycomacropeptide-Containing Food Composition)

Examples of a food composition produced using a production method for a glycomacropeptide-containing food composition of the present invention (hereinafter, may be referred to simply as "food composition") include, for example, general beverages and alcoholic beverages such as carbonated beverages, various fruit juices, fruit juice beverages, refreshing beverages including fruit juices, beverages with bits, fruit beverages with fruit grains, vegetable-based beverages including various vegetables, soybean milk and soybean milk beverages, coffee beverages, tea beverages, powdered beverages, condensed beverages, sports beverages, and nutritional beverages; confectionary such as caramels and candies, chewing gums, chocolates, cookies and biscuits, cakes and pies, snacks and crackers, Japanese confectionary and rice cakes, bean cakes, and dessert sweets; fats and oils such as butter, margarines, mayonnaises, and plant oils; milk and milk products such as bovine milks and processed milks, milk beverages, yogurts, lactic acid bacteria beverages, cheese, ice creams, formula milks, and cream; agricultural processed products such as cereals (grain processed products); and other commercially available foods such as baby foods and liquid diets.

Additives and materials such as an antioxidant, a flavoring ingredient, an acidulant, a coloring agent, an emulsifier, a preservative, a seasoning agent, a sweetener, a spice, a pH adjuster, a stabilizer, a plant oil, an animal oil, sugar and sugar alcohols, a vitamin, an organic acid, fruit juice extracts, vegetable extracts, cereals, beans, vegetables, meats, and fish and seafoods can be blended each alone or in combination of two or more thereof as desired into the food composition. The blending amount of each of the materials and additives can be determined as necessary.

The form of the food composition is not especially limited, and the food composition may take, for example, a liquid form or a fluent form such as that of a beverage or a liquid diet, may take a jelly form, a paste form, a semi-liquid form, or a gel form, or may take a form of a solid piece, a bar, or powder.

The timing for the step of adding the glycomacropeptide-containing composition to the food composition can be adjusted as necessary taking into consideration the type and the like of the food composition. The glycomacropeptide-containing composition may be added to the food composition in the course of producing the food composition, or the glycomacropeptide-containing composition may be added to the food composition after the production of the food composition is completed. The form of the glycomacropeptide-containing composition at the time of the addition thereof is not limited and the glycomacropeptide-containing composition may be added in the state of the permeate, or may be added in the state of the powder after being freeze-dried.

The blending amount of the glycomacropeptide-containing composition to the food composition is not especially limited as long as the blending amount is in a range with which the effects of the present invention are not degraded, and the blending amount only has to be set to include glycomacropeptide in an amount of, for example, 0.01% by weight to 1% by weight or 0.1% by weight to 1% by weight based on the food composition.

The food composition can fill an ordinary packaging container to be provided such as a molded container whose main component is polyethylene terephthalate (a PET bottle), a metal can, a paper container having a metal foil and a plastic film laminated thereon, a pouch such as an aluminum pouch, a plastic container, a vinyl container, a press-through package (PTP), and a bottle such as a glass bottle, and the content volume thereof is not especially limited.

Examples of the food composition include a health food, a functional food, a food with health claims (such as, for example, a food for specified health use, a food with nutrient function claims, and a food with functional claims), a special use food (such as, for example, an infant food, a maternity food, and a patient food), and a nutraceutical food (a supplement).

The food composition may have a presentment of its functions such as, for example, phrases of, "blocks adhesion of pathogens", "strengthens the immune function", "enhances the memory and the learning capacity", "suppresses stresses", "suppresses secretion of IgE", "suppresses inflammation", "suppresses the appetite", "has an antioxidant action", "facilitates absorption of minerals", "increases beneficial bacteria", and "facilitates production of short-chain fatty acids".

### (Microparticulated Whey Composition for Producing Glycomacropeptide-containing Composition)

The present invention provides a microparticulated whey composition (MP whey composition) to produce the glycomacropeptide-containing composition. The MP whey composition of the present invention may be prepared by the user or a commercially available MP whey composition whose volumetric median diameter (d50) is 0.1 µm to 100 µm can be used as the MP whey composition of the present invention. Examples of the commercially available MP whey composition include Y0-8075 (Aria Foods amba) and WPC550 (Fonterra Co-operative Group Limited). In the case where the MP whey composition is solved in water to be sufficiently suspended, the volumetric median diameter (d50) thereof is 0.1 µm to 100 µm, preferably 0.1 µm to 50 µm, more preferably 0.1 µm to 10 µm, further preferably 0.1 µm to 5 µm, and most preferably 0.1 µm to 3 µm.

The whey protein included in cheese whey or rennet casein whey that is acquired in the course of producing a milk product such as cheese may be used or the whey protein included in a whey protein raw material such as a whey protein concentrate (WPC) or a whey protein isolate (WPI) may be used, as the whey protein to prepare the MP whey of the present invention. It is preferred that the cheese whey or the rennet casein whey that is acquired in the course of producing a milk product such as cheese be used. The MP whey composition of the present invention is acquired by heating and applying the shear process to the whey protein raw material. The conditions for the heating and the shear process are as above.

The MP whey composition of the present invention can also be prepared based on a commercially available MP whey composition. In the case where the volumetric median diameter (d50) of the commercially available MP whey composition is larger than 100 µm, an MP whey composition whose volumetric median diameter (d50) is 0.1 µm to 100 µm can be acquired by solving the commercially available MP whey composition in a proper solvent and processing with a homogenizer.

In the case where the volumetric median diameter (d50) of the commercially available MP whey composition is 50 µm to 100 µm, it is also preferred that the commercially available MP whey composition be homogenized to reduce its volumetric median diameter (d50). Mineral whey may be added to and mixed with the MP whey composition of the present invention to supply minerals and the like, and these two may both be thereafter homogenized.

The MP whey composition of the present invention can take an optional form such as, for example, a powder form, a liquid form, or a gel form. The MP whey composition takes preferably a powder form or a liquid form, and more preferably a liquid form.

The content of the MP whey in the MP whey composition of the present invention is, for example, 10% by weight or higher, 15% by weight or higher, 16% by weight or higher, 20% by weight or higher, 30% by weight or higher, 40% by weight or higher, 50% by weight or higher, 60% by weight or higher, 70% by weight or higher, 80% by weight or higher, or 90% by weight or higher, based on the total amount of the composition. The upper limit thereof is 90% by weight or lower, 80% by weight or lower, 70% by weight or lower, 60% by weight or lower, 50% by weight or lower, 40% by weight or lower, 31% by weight or lower, 30% by weight or lower, 20% by weight or lower, or 15% by weight or lower. A specific range is 10% by weight to 90% by weight, 15% by weight to 80% by weight, or 16% by weight to 31% by weight.

The content of the MP whey in the MP whey composition of the present invention is, for example, 10% by weight or higher, 15% by weight or higher, 20% by weight or higher, 30% by weight or higher, 40% by weight or higher, 50% by weight or higher, 60% by weight or higher, 70% by weight or higher, 80% by weight or higher, or 90% by weight or higher, in the protein total amount. The upper limit is 90% by weight or lower, 80% by weight or lower, 70% by weight or lower, 60% by weight or lower, 50% by weight or lower, 40% by weight or lower, 30% by weight or lower, 20% by weight or lower, or 15% by weight or lower, A specific range thereof is 10% by weight to 90% by weight, or 15% by weight to 80% by weight.

### EXAMPLES

Examples of the present invention will be described below in detail while the present invention is not limited thereto. In the case where no description is especially made, "%" indicates "% by weight".

### [Example 1] Preparation of High-GMP Content Material Using Cheddar Cheese Whey as Raw Material

2,000 kg of cheddar cheese whey (protein at 0.6%) at pH 6.1 was maintained at 50°C for 30 minutes to solve lactose crystals thereof.

Microparticles of casein were removed from the whey by a milk clarifier (AMC-100, Iwai Kikai Kogyo Co., Ltd) at 6,500 ×g and a defatting process was thereafter applied to the whey using a separator.

The defatted whey was condensed at 10°C to have a 20-fold concentration of the original concentration using an organic UF membrane whose molecular weight cut off was 10 kDa (HpHT 3838-K131, the membrane area of 5 m², Lenntech) to thereby prepare 100 kg of defatted condensed milk. The composition of the defatted condensed milk included protein at 10.1%, lactose at 4.6%, lipid at 0.1%, and ash at 1.6%.

The temperature was maintained for 5 minutes for the defatted condensed milk to be processed at a circumferential velocity of 30 m/s (the diameter of the rotating body of 52 mm and the number of rotations of 11,000 r/min) using Filmix 56-L (PRIMIX Corporation) after the temperature of the defatted condensed milk reached 90°C, and the defatted condensed milk was thereafter cooled for the temperature thereof to reach the room temperature to thereby prepare 100 kg of an MP whey solution. When the MP whey solution was measured using a laser diffraction-type particle size distribution measuring apparatus, the volumetric median diameter (d50) of the MP whey solution was 0.8 µm.

The MP whey solution was processed using a microfiltration membrane whose pore size was 0.1 µm (manufactured by NGK Insulators, Ltd., CEFILT-MF, the membrane area of 2.0 m²) to acquire 50 kg of an MP whey condensed solution and 50 kg of a permeate.

6.0 kg of powder (a glycomacropeptide-containing composition) acquired by condensing and drying the permeate had a GMP content per protein of 80% by weight.

The MP whey condensed solution acquired as a membrane condensation fraction had no aggregation and no gelation occurring therein and it was considered that the solution was usable for any uses.

### [Example 2] Preparation of High GMP-Content Material Using Camembert Cheese Whey as Raw Material

2,000 kg of Camembert cheese whey (protein at 0.6%) at pH 6.1 was maintained at 50°C for 30 minutes to solve lactose crystals thereof.

Microparticles of casein were removed from the whey by a milk clarifier (AMC-100, Iwai Kikai Kogyo Co., Ltd) at 6,500 ×g and a defatting process was thereafter applied thereto using a separator.

The defatted whey was condensed at 10°C to have a 20-fold concentration of the original concentration using an organic UF membrane whose molecular weight cut off was 10 kDa (HpHT 3838-K131, the membrane area of 5 m², Lenntech) to thereby prepare 100 kg of defatted condensed milk.

The temperature was maintained for 5 minutes for the defatted condensed milk to be processed at a circumferential velocity of 30 m/s (the diameter of the rotating body is 52 mm and the number of revolutions is 11,000 r/min) using Filmix 56-L (PRIMIX Corporation) after the temperature of the defatted condensed milk reached 90°C and the defatted condensed milk was thereafter cooled for the temperature thereof to reach the room temperature to thereby prepare 100 kg of an MP whey solution. When the MP whey solution was measured using a laser diffraction-type particle size distribution measuring apparatus, the volumetric median diameter (d50) of the MP whey solution was 1.3 µm.

The MP whey solution was processed using a microfiltration membrane whose pore size was 0.1 µm (manufactured by NGK Insulators, Ltd., CEFILT-MF, the membrane area of 2.0 m²) to acquire 50 kg of an MP whey condensed solution and 50 kg of a permeate.

6.0 kg of powder (a glycomacropeptide-containing composition) acquired by condensing and drying the permeate had a GMP content per protein of 80% by weight.

The MP whey condensed solution acquired as a membrane condensation fraction had no aggregation and no gelation occurring therein and it was considered that the solution was usable for any uses.

### [Example 3] Preparation of High GMP-Content Material by Diafiltration Using Camembert Cheese Whey as Raw Material

2,000 kg of Camembert cheese whey (protein at 0.6%) at pH 6.1 was maintained at 50°C for 30 minutes to solve lactose crystals thereof.

Microparticles of casein were removed from the whey by a milk clarifier (AMC-100, Iwai Kikai Kogyo Co., Ltd) at 6,500 ×g and a defatting process was thereafter applied thereto using a separator.

The defatted whey was condensed at 10°C to have a 20-fold concentration of the original concentration using an organic UF membrane whose molecular weight cut off was 10 kDa (HpHT 3838-K131, the membrane area of 5 m², Lenntech) to thereby prepare 100 kg of defatted condensed milk. Of the permeate generated as a secondary product of the process (a UF permeate), 100 kg was collected and was used at a diafiltration step executed later.

The temperature was maintained for 5 minutes for the defatted condensed milk to be processed at a circumferential velocity of 30 m/s (the diameter of the rotating body is 52 mm and the number of revolutions is 11,000 r/min) using Filmix 56-L (PRIMIX Corporation) after the temperature of the defatted condensed milk reached 90°C and the defatted condensed milk was thereafter cooled for the temperature thereof to reach the room temperature to thereby prepare 100 kg of an MP whey solution. When the MP whey solution was measured using a laser diffraction-type particle size distribution measuring apparatus, the volumetric median diameter (d50) of the MP whey solution was 1.3 µm.

A diafiltration process was applied to 100 kg of the MP whey solution using a microfiltration membrane whose pore size was 0.1 µm (manufactured by NGK Insulators, Ltd., CEFILT-MF, the membrane area of 2.0 m²) and adding thereto 100 kg of the UF permeate, to acquire 100 kg of a retentate of the MP whey diafiltration and 100 kg of a permeate.

6.0 kg of powder (a glycomacropeptide-containing composition) acquired by condensing and drying the permeate had a GMP content per protein of 80% by weight.

The retentate of the MP whey diafiltration acquired as a membrane condensation fraction of the diafiltration had no aggregation and no gelation occurring therein and it was considered that the retentate was usable for any uses.

### [Example 4] Preparation of High GMP-Content Material Using Commercially Available MP Whey Powder

Y08075 (MP whey powder: Arla Foods amba) was used as the commercially available MP whey powder. Water was added to the powders of Y08075 (final 10% (w/w)) (MP whey powder; Arla Foods amba) and Promilk 102 (final 7.5% (w/w)) (mineral whey powder: Ingredia) and the powder was thereafter sufficiently suspended using TK Homo-Mixer (5,000 rpm, Tokushu Kikai Kogyo Co., Ltd.). The suspended MP whey was next processed using a homogenizing machine (30 MPa, Aluminum, Plant, and Vessel Co., Ltd.) to produce an MP whey solution. When the MP whey solution was measured by a laser diffraction-type particle size distribution measuring apparatus, the volumetric median diameter (d50) of this MP whey solution was 1.3 µm.

The MP whey solution was processed using a microfiltration membrane whose pore size was 0.1 µm (manufactured by NGK Insulators, Ltd., CEFILT-MF, the membrane area of 2.0 m²) to acquire 50 kg of an MP whey condensed solution and 50 kg of a permeate.

6.0 kg of powder (a glycomacropeptide-containing composition) acquired by condensing and drying the permeate had a GMP content per protein of 80% by weight.

The MP whey condensed solution acquired as a membrane condensation fraction had no aggregation and no gelation occurring therein and it was considered that the liquid was usable for any uses.

The volumetric median diameter (d50) of the solution after being suspended by TK Homo-Mixer and before being processed by the homogenizing machine was 58 µm. It is considered that, in the case where this solution underwent the microfiltration membrane process, the condensation, and drying without applying the homogenizing process thereto to acquire powder that included GMP, insoluble matters would precipitate and the membrane might tend to be clogged, but the same effect would be acquired.

### [Example 5] Preparation of High GMP-Content Material Using Camembert Cheese Whey as Raw Material (Centrifugal Separation)

2,000 g of Camembert cheese whey (protein at 0.6%) at pH 6.1 was maintained at 50°C for 30 minutes to solve lactose crystals thereof.

Microparticles of casein were removed from the whey by a milk clarifier (AMC-100, Iwai Kikai Kogyo Co., Ltd) at 6,500 ×g and a defatting process was thereafter applied thereto using a separator.

The defatted whey was condensed at 10°C to have a 20-fold concentration of the original concentration using an organic UF membrane whose molecular weight cut off was 10 kDa (HpHT 3838-K131, the membrane area of 5 m², Lenntech) to thereby prepare 100 kg of defatted condensed milk.

The temperature was maintained for 5 minutes for the defatted condensed milk to be processed at a circumferential velocity of 30 m/s (the diameter of the rotating body is 52 mm and the number of revolutions is 11,000 r/min) using Filmix 56-L (PRIMIX Corporation) after the temperature of the defatted condensed milk reached 90°C and the defatted condensed milk was thereafter cooled for the temperature thereof to reach the room temperature to thereby prepare 100 kg of an MP whey solution. When the MP whey solution was measured using a laser diffraction-type particle size distribution measuring apparatus, the volumetric median diameter (d50) of the MP whey solution was 1.3 µm.

The MP whey solution was processed at 8,000 rpm, with a supply flow of 165 L/h, and at the pressure on the heavy liquid side of 0.27 MPa using a milk separator (SMP-500PS, manufactured by Saito Separator Limited) to acquire 82 kg of a heavy liquid including the MP whey and 18 kg of a light liquid.

2.2 kg of powder (a glycomacropeptide-containing composition) acquired by condensing and drying the light liquid had a GMP content per protein of 20% by weight.

The MP whey condensed solution acquired on the heavy liquid side had no aggregation and no gelation occurring therein and it was considered that the solution was usable for any uses.

### [Example 6] Preparation of High GMP-Content Material Using Gouda Cheese Whey as Raw Material

2,000 kg of Gouda cheese whey (protein at 0.7%) at pH 6.2 was maintained at 50°C for 30 minutes to solve lactose crystals thereof.

Microparticles of casein were removed from the whey by a milk clarifier (AMC-100, Iwai Kikai Kogyo Co., Ltd) at 6,500 ×g and a defatting process was thereafter applied thereto using a separator.

The defatted whey was condensed at 10°C to have a 20-fold concentration of the original concentration using an organic UF membrane whose molecular weight cut off was 10 kDa (HpHT 3838-K131, the membrane area of 5 m², Lenntech) to thereby prepare 100 kg of defatted condensed milk.

The temperature was maintained for 5 minutes for the defatted condensed milk to be processed at a circumferential velocity of 30 m/s (the diameter of the rotating body is 52 mm and the number of revolutions is 11,000 r/min) using Filmix 56-L (PRIMIX Corporation) after the temperature of the defatted condensed milk reached 90°C and the defatted condensed milk was thereafter cooled for the temperature thereof to reach the room temperature to thereby prepare 100 kg of an MP whey solution. When the MP whey solution was measured using a laser diffraction-type particle size distribution measuring apparatus, the volumetric median diameter (d50) of the MP whey solution was 1.1 µm.

The MP whey solution was processed using an ultrafiltration membrane whose molecular weight cut off was 100,000 Da (manufactured by DDS, GR40PP, the membrane area of 3 m²) to acquire 50 kg of an MP whey condensed solution and 50 kg of a permeate.

5.3 kg of powder (a glycomacropeptide-containing composition) acquired by condensing and drying the permeate had a GMP content per protein of 80% by weight.

The MP whey condensed solution acquired as a membrane condensation fraction had no aggregation and no gelation occurring therein and it was considered that the solution was usable for any uses.

### [Example 7] Recondensation and Freeze-Drying

The permeate acquired in Example 1 was recondensed to have a solid content at 60% using a vacuum concentration machine and lactose thereof was crystallized at 2°C to be removed. The crystallized lactose was washed with water and 10 kg of the acquired mother liquid was creese-dried to acquire 1.2 kg of powder (a glycomacropeptide-containing composition).

### [Example 8] Recondensation and Freeze-Drying

The permeate acquired in Example 6 was recondensed using an ultrafiltration membrane whose molecular weight cut off was 8,000 Da (the membrane area of 4 m², manufactured by Abcor Japan Co., Ltd., HF K-131) and the acquired liquid that was recondensed was demineralized by diafiltration. 25 kg of the acquired demineralized condensed liquid was spray-dried to acquire 1.0 kg of powder (a glycomacropeptide-containing composition).

### [Example 9] Production of Food Composition (Cheese)

15.0 kg of raw milk was homogenized and was thereafter sterilized at 63°C for 30 minutes. A starter was added to the homogenized and sterilized raw milk to react with each other for about 2 hours. After the reaction, the condensed liquid acquired in Example 1 was added to the raw milk at 1.0% by weight, and calcium chloride, white mold, and rennet were further added thereto to react the substances with each other for about 1 hour. After cutting the reaction product, the product was packed in a mold. After removing the whey, the product was immersed in salt water. The product then underwent primary ripening and packaging. The product then underwent secondary ripening to obtain Camembert cheese.

### INDUSTRIAL APPLICABILITY

According to the present invention, a production method for a novel high-GMP content composition is provided. The MP whey collected on the condensed liquid side by the membrane process is not different compared to an ordinary MP whey to which no membrane process is applied except the fact that the former has an increased protein content. The MP whey collected on the condensed liquid side is usable for various foods as an ordinary MP whey. As above, both a high GMP-content composition and the usable MP whey can be prepared from an ordinary MP whey.

## Claims

1. A production method for a glycomacropeptide-containing composition, comprising
condensing and/or applying diafiltration to a microparticulated whey solution, and
acquiring a solution containing glycomacropeptide,
wherein a glycomacropeptide content in a protein total amount is 10% by weight or higher.

2. The production method for a glycomacropeptide-containing composition according to claim 1, wherein
the condensation and/or the diafiltration is/are each a microfiltration membrane process, an ultrafiltration membrane process, an ion exchanging membrane process, or centrifugal separation.

3. The production method for a glycomacropeptide-containing composition according to claim 2, wherein
the condensation and/or the diafiltration is/are each the microfiltration membrane process or the ultrafiltration membrane process,
the membrane is a microfiltration membrane whose pore size is 10 µm or smaller or an ultrafiltration membrane whose molecular weight cut off is 50,000 Da or higher, and
a glycomacropeptide-containing composition is acquired as a permeate.

4. The production method for a glycomacropeptide-containing composition according to any one of claims 1 to 3, further comprising
acquiring a microparticulated whey solution by heating and applying a shear process to a whey protein solution.

5. The production method according to any one of claims 1 to 4, wherein
a volumetric median diameter (d50) of the microparticulated whey is 0.1 µm to 100 µm.

6. The production method for a glycomacropeptide-containing composition according to any one of claims 1 to 5, further comprising
removing lactose and/or minerals by recondensing, applying rediafiltration to, and/or demineralizing to the solution containing glycomacropeptide.

7. The production method for a glycomacropeptide-containing composition according to claims 6, wherein
the removal of the lactose and/or the minerals is executed using an ultrafiltration membrane whose molecular weight cut off is 10,000 Da or lower, or vacuum concentration.

8. A production method for a glycomacropeptide-containing composition, comprising
condensing, applying diafiltration to, and/or demineralizing the composition acquired by the production method for a glycomacropeptide-containing composition according to any one of claims 1 to 7, and
removing lactose and minerals from the composition

9. A microparticulated whey composition for producing a glycomacropeptide-containing composition .

10. A production method for a glycomacropeptide-containing food composition, comprising:
producing a glycomacropeptide-containing composition by the production method for a glycomacropeptide-containing composition according to any one of claims 1 to 8; and
adding the glycomacropeptide-containing composition to a food.
